# EUROPEAN PATENT APPLICATION

(11) **EP 3 828 893 A1**
(43) Date of publication of application: **02.06.2021**
(21) Application number: 19218126.1
(22) Date of filing: 19.12.2019
(51) Int. Cl.: G16C 20/70, G06N 20/00

(54) **APPARATUS AND METHOD FOR CONSTRUCTING LIBRARY FOR DERIVING MATERIAL COMPOSITION**

(30) Priority: 29.11.2019 KR 20190157515
(71) Applicant: Korea Advanced Institute Of Science And Technology, Daejeon, 34141 (KR)
(72) Inventor: HONG, Seung Bum, 34141 Daejeon (KR); CHO, Eun Ae, 34141 Daejeon (KR); YUK, Jong Min, 34141 Daejeon (KR); BYON, Hye Ryung, 34141 Daejeon (KR); YANG, Yong Soo, 34141 Daejeon (KR); CHOI, Pyuck Pa, 34141 Daejeon (KR); SHIN, Jong Hwa, 34141 Daejeon (KR); LEE, Hyuck Mo, 34141 Daejeon (KR); LIOW, Chi Hao, 34141 Daejeon (KR); CHO, Seong Woo, 34141 Daejeon (KR); PARK, Gun, 34141 Daejeon (KR); LEE, Yong Ju, 34141 Daejeon (KR); SHIM, Yoon Su, 34141 Daejeon (KR); NA, Moo Ny, 34141 Daejeon (KR); JUN, Ho Sun, 34141 Daejeon (KR); BANG, Ki Hoon, 34141 Daejeon (KR); KIM, Myung Joon, 34141 Daejeon (KR); JEONG, Chae Hwa, 34141 Daejeon (KR); KIM, Seung Gu, 34141 Daejeon (KR); OH, Chung Ik, 34141 Daejeon (KR); KIM, Hong Jun, 34141 Daejeon (KR); KIM, Jae Gyu, 34141 Daejeon (KR); OH, Ji Min, 34141 Daejeon (KR); YEOM, Ji Won, 34141 Daejeon (KR); EOM, Seong Mun, 34141 Daejeon (KR); KIM, Hyoung Kyu, 34141 Daejeon (KR); HAN, Young Joon, 34141 Daejeon (KR); LEE, Dae Hee, 34141 Daejeon (KR); LEE, Ho Jun, 34141 Daejeon (KR); KIM, Jae Woon, 34141 Daejeon (KR); SHIN, Jae Wook, 34141 Daejeon (KR); KANG, Hyeon Muk, 34141 Daejeon (KR); PARK, Jae Yeol, 34141 Daejeon (KR); JEONG, Han Beom, 34141 Daejeon (KR); LEE, Jae Sang, 34141 Daejeon (KR); CHANG, Joon Ha, 34141 Daejeon (KR); KIM, Yo Han, 34141 Daejeon (KR); KIM, Su Jung, 34141 Daejeon (KR); OH, Hyun Jeong, 34141 Daejeon (KR); BAUCOUR, Arthur, 34141 Daejeon (KR); HAN, Jae Wook, 34141 Daejeon (KR); JANG, Kyu Seon, 34141 Daejeon (KR); JO, Hye Sung, 34141 Daejeon (KR); YOO, Bo Ryung, 34141 Daejeon (KR); PARK, Hyeon Jin, 34141 Daejeon (KR); CHO, Min Gwan, 34141 Daejeon (KR); PARK, Jun Hyung, 34141 Daejeon (KR); KIM, Yea Eun, 34141 Daejeon (KR); MIN, Seok Hwan, 34141 Daejeon (KR); CHOI Jung Woo, 34141 Daejeon (KR); PARK, Young Tae, 34141 Daejeon (KR); HONG, Doo Sun, 34141 Daejeon (KR)
(74) Representative: Zimmermann, Tankred Klaus

(57) **Abstract**

The present invention provides an apparatus and a method for constructing a library for deriving a material composition using empirical result. Which enables acceleration of research on the material-properties relationship. By applying the empirical results of the material composition, missing data of the material compositions can be statistically calculated by using supervised non-linear imputation techniques. The completed composition information of the materials is passed as an input of machine learning material-properties relationship prediction.

## Description

### BACKGROUND OF THE INVENTION

### Field of the Invention

The present invention relates to an apparatus and a method for finding the optimum composition of a material having a target performance, and more particularly, an apparatus and a method for constructing a library using empirical or experimental result to impute missing data by supervised non-linear imputation techniques, which enables acceleration of research on the material.

### Description of the Related Art

When trying to develop a material with a target performance, predicting the composition of the material for the target performance enables a fast and accurate research.

Without a doubt, existing experiments and theoretical calculations based on a trial-and-error method provide insightful knowledge and allow users to discover new materials and interesting properties. However, the disadvantage of such an approach is that it is time consuming and cost inefficient.

Recently, the machine learning with prior knowledge of a correlation between input functions and attributes of interest provides a flexible and accessible framework that reduces the time taken for trial and error and getting accurate results.

The machine learning has been widely applied in the field of energy materials such as batteries, and the calculations so far have depended heavily on theoretical input functions. For example, it is possible to input a desired electrode configuration as input data of the machine learning and calculate an output voltage. Another approach showed that the machine learning can calculate properties of candidate materials on the basis of small amount of input data in a physical equation guide models.

However, since the input function is highly dependent on theoretical parameters, many other factors that may affect the performance of the material system may not be captured, which causes a problem of occurrence of inconsistency between the calculated results and the empirical results.

In the case of a battery, which is an example of energy materials, patents such as US 2016/0363632 A1 and US 9,774,203 B2 relating to the use of machine learning put emphasis on monitoring of the battery state such as a deterioration state, an optimal heating condition for a battery lead time, a life recyclability of a rechargeable battery and a life prediction of the battery.

There are only few reports that use the machine learning as a research tool to find the optimal operating conditions in the batteries. Recently, it has been proved that optimal manufacturing conditions for better battery performance can be found by using experimental conditions such as composition, sintering temperature, type and amount of dopant, cleaning condition, coating materials as input features. However, since this takes only the inherent attributes of the battery into account and ignores external contributions during battery operation, there is a problem of an increase in deviation between the result and the actually measured value.

### Citation List

### Patent Literature

Patent Literature 1: US 2016/0363632 A1
Patent Literature 2: US 9,774,203 B2

### SUMMARY OF THE INVENTION

Therefore, embodiments of the present invention for solving the above-mentioned problems of the related art provide an apparatus and a method for constructing a library for deriving material composition and deriving material composition to accelerate the research on the material development, by using empirical result including intrinsic and extrinsic attributes on the basis of experimental results. The apparatus and a method of the present invention derive the missing data of the experimental results obtained from patents or journal publications using supervised non-linear imputation technique, and construct complete dataset including intrinsic and extrinsic attributes for deriving material composition, and derive the library and material composition by applying the complete dataset as input data of the machine learning.

According to an embodiment of the present invention for achieving the above-described object, the apparatus for constructing a library for deriving a material composition including:
an empirical result including a missing value preprocessing unit which classifies empirical result including a missing value result as parameter correlated with features of a material to be developed and constructs an empirical result set including the missing value;
a completed empirical result sets deriving unit which derives parameters corresponding to the missing values by applying a supervised non-linear imputation technique to the empirical result including the classified missing value, and, derive the completed empirical result sets by imputing the parameters to the he missing values;
an optimization parameter deriving unit which derives optimized hyperparameters among the parameters included in the completed empirical result sets; and
a material composition library constructing unit, which calculates feature values of the material by machine learning which takes the completed empirical result sets having the derived optimized hyperparameters as an input, to construct a material composition library.

According to another embodiment of the present invention for achieving the above-described object, there is provided a method for constructing a library for deriving a material composition, the method including:
an empirical result including a missing value classifying step of classifying empirical result including a missing value result as parameter correlated with features of a material to be developed and constructs an empirical result set including the missing value;
a completed empirical result sets deriving step of deriving parameters corresponding to the missing values by applying a supervised non-linear imputation technique to the empirical result including the classified missing value, and, derive the completed empirical result sets by imputing the parameters to the he missing values;
an optimization parameter deriving step of deriving optimized hyperparameters among the parameters included in the completed empirical result sets; and
a material composition library constructing step of calculating feature values of the material by executing machine learning which takes the completed empirical result sets having the derived optimized hyperparameters as an input to construct a material composition library.

The missing values may be omitted parameters among the parameters of the material included in the empirical result.

The empirical result may be a material-related gathered data with parameters correlated to the feature value of the material included in one or more of material-related patents, theses, and research literatures.

The parameter may include one or more of a starting material, structure crystallite, physical properties, and measurement conditions of the material.

The supervised non-linear imputation technique may be an imputation algorithm having multiple imputation including one or more of Random Forest (RF), K-nearest neighbors (KNN) or multiple imputation by chained equations (MICE).

The optimization of hyperparameter may apply a search-grid optimization method to derive optimized hyperparameters on the completed empirical result sets.

### BRIEF DESCRIPTION OF THE DRAWINGS

FIG. 1 is a functional block diagram of an apparatus 1 for constructing a library of a derived material composition according to an embodiment of the present invention;
FIG. 2 is a flowchart showing a process of a method for constructing a library of a derived material composition according to another embodiment of the present invention;
FIG. 3 is a diagram showing a process of constructing a library for deriving a cathode material of a lithium battery as an embodiment of the present invention;
FIG. 4 is a diagram showing a Pearson correlation map of input features and capacity target output of the empirical result of a lithium battery cathode material based on the imputed data calculated using (a) Random Forest (b) KNN (C) MICE;
FIG. 5 is a graph showing a composition-capacity distribution of NCM-based lithium battery material using libraries derived by applying the empirical result of the NCM-based lithium battery material of an embodiment of the present invention to machine learning;
FIG. 6(a) is an SEM micrograph of NCM (0.5 0.3 0.2)-based lithium battery cathode material particles, and FIG. 6(b) is an enlarged SEM micrograph of the NCM (0.5 0.3 0.2)-based lithium battery cathode material, which are manufactured by applying the library constructed according to an embodiment of the present invention; and
FIG. 7 is a graph that shows a comparison between predicted and actual charge capacities of the lithium battery cathode material predicted on the basis of the completed empirical result sets constructed by (a) Random Forest (b) KNN (C) MICE during execution of the process of FIG. 3.

### DETAILED DESCRIPTION OF THE PREFERRED EMBODIMENTS

Hereinafter, the present invention will be described with reference to the accompanying drawings. However, the present invention can be embodied in various different forms and is therefore not limited to the embodiments described herein. In order to clearly describe the present invention in the drawings, parts not related to the description are omitted, and similar parts are denoted by similar reference numerals throughout the specification.

Throughout the specification, if some parts are "coupled (connected, contacted, combined)" with other parts, this includes not only a case of being "directly connected", but a case of being "indirectly connected" with another member between the parts. Also, if any part "includes" any component, it means that other components can be further included rather than excluding other components, unless otherwise stated.

The terms used herein are merely for the purpose of describing particular embodiments and are not intended to limit the invention. A singular expression includes a plural expression unless clearly otherwise stated. As used herein, it should be understood that terms such as "including" or "having" are intended to designate existence of feature, number, step, operation, component, part or combinations thereof described in the specification, but are not intended to exclude the existence or additional possibilities of one or more other features or numbers, steps, operations, components, parts or combinations thereof in advance.

Hereinafter, embodiments of the present invention will be described in detail with reference to the accompanying drawings.

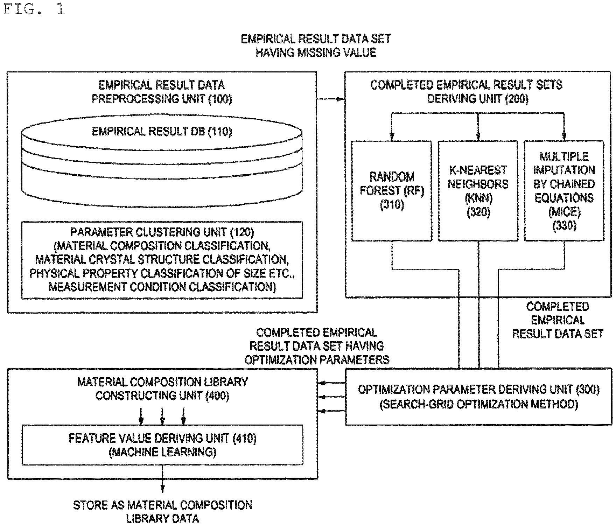

FIG. 1 is a functional block diagram of an apparatus 1 for constructing a library for deriving a material composition according to an embodiment of the present invention.

As shown in FIG. 1, the apparatus 1 for constructing the library for deriving the material composition using empirical result of the material composition (hereinafter referred to as an "apparatus 1 for constructing a library for deriving a material composition") of an embodiment of the present invention may be configured to include an empirical result preprocessing unit 100, a completed empirical result sets deriving unit 200, an optimization parameter deriving unit 300, and a material composition library constructing unit 400.

The empirical result preprocessing unit 100 is configured to classify the empirical result including missing values, which are parameters, omitted from the empirical result which parameter correlated with features of a material to be developed, and construct an empirical result set including the missing values.

In the aforementioned configuration, the empirical result means material-related accumulated data where parameters correlated with a feature value of the material included in one or more of material-related patents, theses, and research literatures.

In order to execute the aforementioned function, the empirical result preprocessing unit 100 is configured to include empirical resultbase (DB) 110 that stores material-related accumulated data having parameters correlated with the feature values of the material included in one or more of the material-related patents, theses, and research literatures as empirical result. A parameter clustering unit 120 that constructs an empirical result set including the missing value having the missing value as a parameter from which the empirical result are missing, depending on the parameters of the material.

The empirical result may be data collected by constructing a search engine.

The parameters of the material may include one or more of starting materials, structure crystallite, physical properties or measurement conditions. Specifically, the aforementioned parameters may include experimental conditions such as composition, sintering temperature, dopant, cleaning, coating, an ICP and a XRD.

As an example of a standard for extracting empirical result including the missing value, in the case of developing a lithium battery, it may having a three or less components without doping. The empirical result, including the extracted missing values, are clustered depending on the parameters such as a composition of the starting material, a heat treatment temperature and time for establishing the crystal structure, a particle size of the physical features, an output voltage or a discharge rate (C-rate) measurement condition as the material feature value.

The completed empirical result sets deriving unit 200 is configured to derive the missing values by applying the supervised non-linear imputation technique to the empirical result including the classified missing value, and then, impute the missing values and derive data sets to form a completed experimental result having parameters with no missing value.

For the application of the supervised non-linear imputation technique for the derivation of the parameters corresponding to the missing values, the completed empirical result sets deriving unit 200 is configured to execute multiple imputation algorithm that processes the multiple imputations in parallel by taking the empirical result sets including the missing value as an input.

As an example of the multiple imputation algorithm, as shown in FIG. 1, an imputation algorithm having multiple imputations including one or more of Random Forest (RF), K-nearest neighbors (KNN) or Multiple imputation by chained equations (MICE) which indicated in unit 200.

The Random Forest algorithm imputes missing values in accordance with non-linear interactions in the parameters. The K-nearest neighbor algorithm looks for omitted data (parameters) on the basis of the nearest k neighbors. The MICE imputes missing values on the basis of the conditional variable distribution model. All the above three methods operate in a multidimensional space rather than a single imputation. In this way, the output voltage and charge capacity can be calculated using a trained machine learning model with data set having no missing value, in which the missing value is imputed, as an input function.

The optimization parameter deriving unit 300 is configured to derive optimized hyperparameters among the parameters included in the completed empirical result sets. The optimized hyperparameters derived from the optimization parameter deriving unit 300 are parameters having a correlation with the target feature value of the material more than a predetermined correlation. As an example, the optimized hyperparameters may be calculated by applying a search-grid optimization method.

The material composition library constructing unit 400 is a configuration to calculate the feature value of the material by the machine learning which takes the completed empirical result sets having the derived optimized hyperparameters as an input, and then construct a material composition library by performing a demonstration comparison on the calculated feature values.

To this end, the material composition library constructing unit 400 equipped with a feature value deriving unit 410. Through the machine learning the completed empirical result having the optimized hyperparameters as an input, and registers the derived material composition and the feature value data and the completed empirical result are used to calculate the target performance and form a library for deriving the material composition.

As mentioned above, the libraries for deriving the material composition derived allows the material feature value, for example, the output voltage or the charging capacity when the material is a lithium battery, to be quickly mapped, on the basis of a compound ratio of the material of interest and the optimal operating conditions.

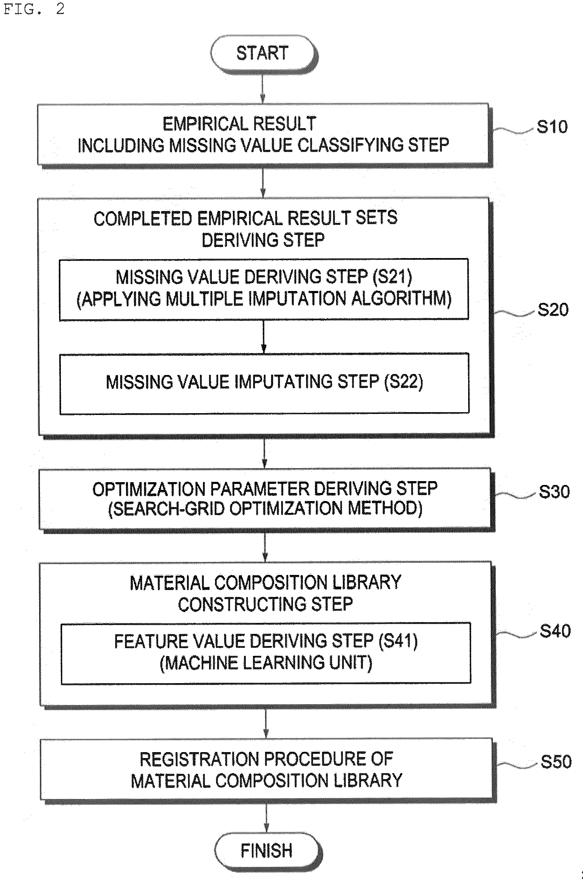

FIG. 2 is a flowchart showing a process of a method for constructing a library by deriving a material composition according to another embodiment of the present invention.

As shown in FIG. 2, the method for constructing the material composition library using the apparatus 1 for constructing the library for deriving the material composition configured to include the empirical result preprocessing unit 100, the completed empirical result sets deriving unit 200, the optimization parameter deriving unit 300, and the material composition library constructing unit 400 may be configured to include an empirical result including a missing value classifying step (S10) executed by the empirical result preprocessing unit 100, a completed empirical result sets deriving step (S20) executed by the completed empirical result sets deriving unit 200, an optimization parameter deriving step (S30) executed by the optimization parameter deriving unit 300, a material composition library constructing step (S40) executed by the material composition library constructing unit 400, and a material composition library registering step (S50).

The empirical result including the missing value classifying step (S10) executed by the empirical result preprocessing unit 100 executes a process of classifying the empirical result including the missing value, which is a parameter, omitted from the empirical result having the parameter correlated with the features of the material to be developed to construct an empirical result set including the missing value.

The empirical result may be material-related accumulated data having parameters correlated with the feature value of the material included in one or more of the material-related patents, theses and research literatures.

The parameters of materials may include one or more of the starting materials, structure crystallite, physical properties, and measurement conditions of the material.

The completed empirical result sets deriving step (S20) executed by the completed empirical result sets deriving unit 200 executes a process of deriving parameters corresponding to the missing values by applying a supervised non-linear imputation technique to the empirical result including the classified missing value, and then imputing the missing values to derive a completed empirical result sets.

The supervised non-linear imputation technique of the completed empirical result sets deriving step (S20) may be an imputation algorithm including one or more of a Random Forest (RF), K-nearest neighbors (KNN) or a Multiple imputation by chained equations (MICE).

The optimization parameter deriving step (S30) executed by the optimization parameter deriving unit 300 executes a process of deriving optimized hyperparameters among the parameters included in the completed empirical result sets. The optimized hyperparameters derived from the optimization parameter deriving unit 300 may be parameters having a correlation with the target feature value of the material more than a predetermined correlation. The optimized hyperparameters having the aforementioned features are derived into optimized hyperparameters of the completed empirical result sets by applying a search-grid optimization method.

The material composition library constructing step (S40) executed by the material composition library constructing unit 400 executes a process of calculating the feature values of the material by the machine learning, which takes the completed empirical result sets having the derived optimized hyperparameters as an input, and registering the material composition and the calculated feature values as a library for deriving a material composition after constructing the material composition library.

### <Example>

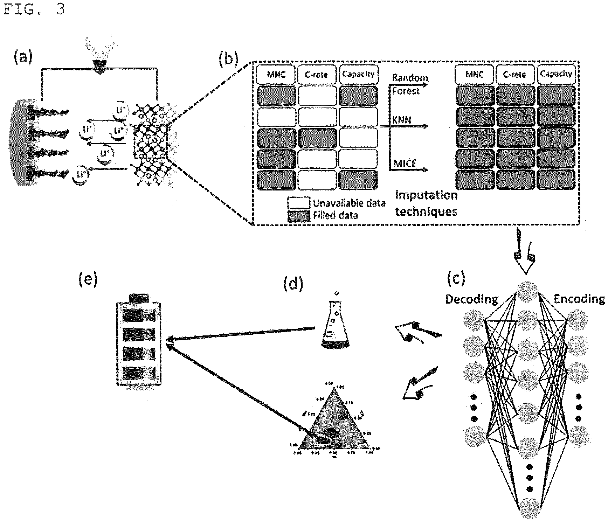

FIG. 3 is a diagram showing a process of constructing a library for deriving a NCM (Ni-Co-Mn)-based lithium battery cathode material as an embodiment of the invention.

As shown in FIG. 3, when a library for deriving an NCM-based lithium battery composition is constructed by applying an embodiment of the present invention, the empirical result including the missing value classifying step (S10) executed by the empirical result preprocessing unit 100 clusters the starting material, the structure crystallite, the physical properties and the measurement condition parameters from the empirical result on the NCM-based lithium battery cathode materials collected on the basis of the NCM-based lithium battery-related patents and theses as shown in FIG. 3(a) and 3(b), and classifies data of three or less components with no doping into the sets of the empirical result having random missing values on the NCM-based lithium battery cathode material.

As shown in FIG. 3(c), the completed empirical result sets deriving step (S20) executed by the completed empirical result sets deriving unit 200 drives and imputes the missing values by the use of a correlation with available value on the basis of the clustering function by applying the empirical result sets of the NCM-based lithium battery cathode material having the classified random missing value to the imputation algorithm having the multiple imputation including Random Forest (RF), K-nearest neighbors (KNN), and Multiple imputation chained equations (MICE), which are supervised non-linear imputation techniques, thereby constructing the completed empirical result sets.

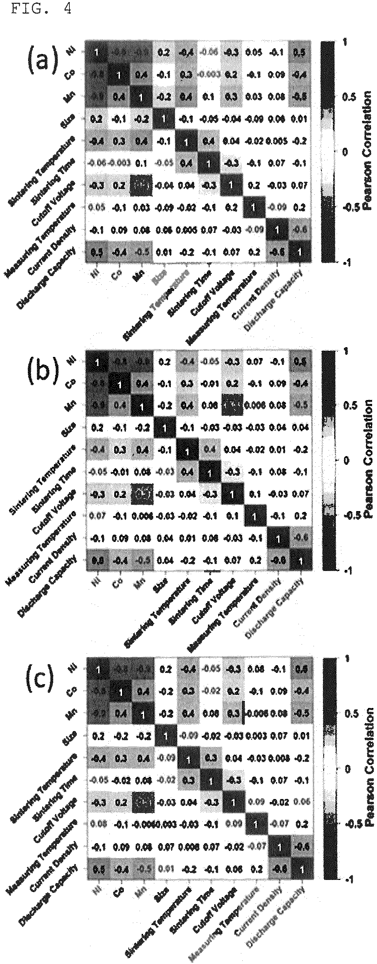

FIG. 4 is a diagram which shows a correlation map of Pearson correlation of input feature and capacity target output of the empirical result of the lithium battery cathode material based on the imputed data calculated using (a) Random Forest (b) KNN (c) MICE.

The optimization parameter deriving step (S30) executed by the optimization parameter deriving unit 300 executes a process of deriving optimized hyperparameters among the parameters included in the completed empirical result sets for the NCM-based lithium battery cathode material. The optimized hyperparameters derived from the optimization parameter deriving unit 300 may be parameters having a correlation with the target feature value of the NCM-based lithium battery cathode material more than a predetermined correlation. The optimized hyperparameters of the empirical result of the NCM-based lithium battery cathode material having the aforementioned features are derived into optimized hyperparameters of the completed empirical result sets by applying a search-grid optimization method.

As shown in FIG. 3(d), the material composition library constructing step (S40) executed by the material composition library constructing unit 400 calculates the feature values of the NCM-based lithium battery cathode material by applying the machine learning, in which the completed empirical result sets having the optimized hyperparameters derived from the completed empirical result of the NCM-based lithium battery cathode material is taken as input, and then, constructs the NCM-based lithium battery cathode material of the composition library and the calculated feature values.

The material composition library constructing step (S40) may execute a demonstration procedure which compares the feature value calculated by selecting a specific sample with the measured value of the actual manufactured conditions of lithium battery cathode. After construction of the lithium battery cathode material composition library and checks whether to have a preset deviation range, for example, a deviation within 10%.

The feature values derived in the feature value deriving step may include a capacity and an average voltage output or a discharge rate (C-rate) of the NCM-based lithium battery cathode material manufactured to have the composition and parameters of the completed data sets derived for the NCM-based lithium battery cathode material.

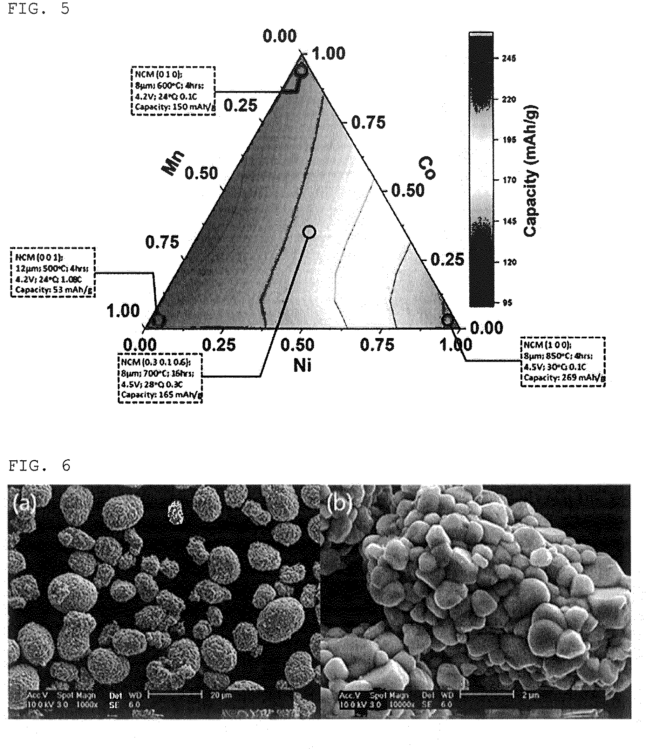

FIG. 5 is a graph that shows the capacity distribution for each composition of the NCM-based lithium battery material using libraries derived by applying the empirical result of the NCM-based lithium battery material of an embodiment of the present invention to the machine learning.

The completed empirical result having the optimized hyperparameters can be selected as a library for deriving the NCM-based lithium battery cathode material composition through the table of FIG. 5. As a result the feature values after manufacturing the NCM-based lithium battery cathode material having the NCM-based lithium battery cathode material composition thus selected, it was checked that the deviation was equal to or less than 10% from the predicted value.

Next, as shown in FIG. 3(e), the material composition library registering step (S50) executes a process of registering the constructed NCM-based lithium battery cathode material composition library as a library for deriving the NCM-based lithium battery cathode material composition.

FIG. 6 is a graph that shows the capacity distribution for each composition of the NCM-based lithium battery cathode material using the libraries derived by applying the empirical result for the NCM-based lithium battery cathode material of an embodiment of the present invention to the machine learning.

FIG. 6(a) is an SEM micrograph of NCM (0.5 0.3 0.2)-based lithium battery cathode material particles, and FIG. 6(b) is an enlarged SEM micrograph of the NCM (0.5 0.3 0.2)-based lithium battery cathode material, which are manufactured by applying the library constructed according to an embodiment of the present invention.

In the case of FIG. 6, the NCM-based lithium battery cathode material was manufactured using FIG. 5 by selecting the composition of NCM, that is, the composition ratio of Ni, Co, and Mn to be 0.5, 0.3, and 0.2. It can be seen through SEM photographs that the electrode production performs smoothly.

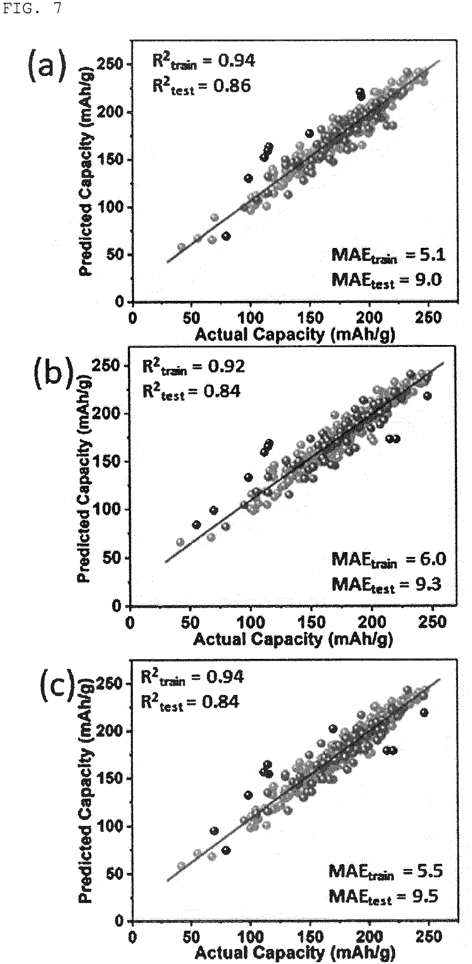

FIG. 7 is a graph that shows a comparison between predicted and actual charge capacities of the lithium battery cathode material predicted on the basis of the completed empirical result sets constructed by (a) Random Forest (b) KNN (C) MICE during execution of the process of FIG. 3

As shown in the graph of FIG. 7, when applying the apparatus and method for constructing the library for deriving the material composition of the present invention, it was checked that the deviation between the predicted feature value of the derived NCM-based lithium battery cathode material and the measured feature value of the actually fabricated NCM-based lithium battery cathode material was 10% or less. It can be seen that, as a value of a linear regression analysis coefficient of determination R2 value is close to 1, a correlation between factors derived through the machine learning is effective, and it can be confirmed that the error is small through the mean error rate (MAE) of less than 10%.

The above-described embodiments of the present invention can also be provided as a system that imputes the missing values of the parameters included in the empirical result of the material, and uses them as input features of machine learning to predict their performances depending on different inherent features and external features. Further, the above-described embodiments of the present invention apply the input function of the machine learning on the basis of the empirical result, thereby narrow a gap between the calculation result and the experiment result in comparison with the input function based on the theoretical data set.

The library for deriving the material composition constructed by the present invention described above provides effects that enable calculation of a relation between each input function and desired output by the used of Pearson correlation analysis, quick grasp of basic functions to maximize the performance of the material, and fast and accurate derivation of the composition of the material to be developed having the target feature value, by the use of a complete empirical result set of materials.

While the present invention has been described with respect to the specific embodiments, it will be apparent to those skilled in the art that various changes and modifications may be made without departing from the spirit and scope of the invention as defined in the following claims.

## Claims

1. An apparatus for constructing a library for deriving a material composition including:
an empirical result including a missing value preprocessing unit which classifies empirical result including a missing value result as parameter correlated with features of a material to be developed and constructs an empirical result set including the missing value;
a completed empirical result sets deriving unit which derives parameters corresponding to the missing values by applying a supervised non-linear imputation technique to the empirical result including the classified missing value, and, derive the completed empirical result sets by imputing the parameters to the he missing values;
an optimization parameter deriving unit which derives optimized hyperparameters among the parameters included in the completed empirical result sets; and
a material composition library constructing unit, which calculates feature values of the material by machine learning which takes the completed empirical result sets having the derived optimized hyperparameters as an input, to construct a material composition library.

2. The apparatus for constructing the library for deriving the material composition according to claim 1, wherein the missing values are omitted parameters among the parameters of the material included in the empirical result.

3. The apparatus for constructing the library for deriving the material composition according to claim 1, wherein the empirical result is a material-related accumulated data having parameters correlated with the feature value of the material included in one or more of material-related patents, theses and research literatures.

4. The apparatus for constructing the library for deriving the material composition according to claim 1, wherein the parameter includes one or more of a starting material, structure crystallite, physical properties, and measurement conditions of the material.

5. The apparatus for constructing the library for deriving the material composition according to claim 1, wherein the supervised non-linear imputation technique is an imputation algorithm having multiple imputation including one or more of Random Forest (RF), K-nearest neighbors (KNN) or Multiple imputation by chained equations (MICE) processed in parallel with each other.

6. The apparatus for constructing the library for deriving the material composition according to claim 1, wherein the optimization parameters are parameters having a correlation with a target feature value of the material more than a predetermined correlation.

7. The apparatus for constructing the library for deriving the material composition according to claim 1, wherein the optimization hyperparameter applies a search-grid optimization method to derive optimized hyperparameters of the completed empirical result sets.

8. method for constructing a library for deriving a material composition, the method including:
an empirical result including a missing value classifying step of classifying empirical result including a missing value result as parameter correlated with features of a material to be developed and constructs an empirical result set including the missing value;
a completed empirical result sets deriving step of deriving parameters corresponding to the missing values by applying a supervised non-linear imputation technique to the empirical result including the classified missing value, and, derive the completed empirical result sets by imputing the parameters to the he missing values;
an optimization parameter deriving step of deriving optimized hyperparameters among the parameters included in the completed empirical result sets; and
a material composition library constructing step of calculating feature values of the material by executing machine learning which takes the completed empirical result sets having the derived optimized hyperparameters as an input to construct a material composition library.

9. The method for constructing the library for deriving the material composition according to claim 8, wherein the missing values of the empirical result classifying step are omitted parameters among the parameters of the material included in the empirical result.

10. The method for constructing the library for deriving the material composition according to claim 8, wherein the empirical result in the empirical result including the missing value classifying step is a material-related gathered data with parameters correlated to the feature value of the material included in one or more of material-related patents, theses, and research literatures.

11. The method for constructing the library for deriving the material composition according to claim 8, wherein the parameter includes one or more of a starting material, structure crystallite, physical features, and measurement conditions of the material.

12. The method for constructing the library for deriving the material composition according to claim 8, wherein the supervised non-linear imputation technique of the completed empirical result sets deriving step is an imputation algorithm having multiple imputation including one or more of Random Forest (RF), K-nearest neighbors (KNN) or multiple imputation by chained equations (MICE).

13. The method for constructing the library for deriving the material composition according to claim 8, wherein the optimization hyperparameters derived in the optimization parameter deriving step are parameters having a correlation with a target feature value of the material greater more than a predetermined correlation.

14. The method for constructing the library for deriving the material composition according to claim 8, wherein the optimization hyperparameters derived in the optimization parameter deriving step are derived into optimized hyperparameters of the completed empirical result sets by applying a search-grid optimization method.
